# EUROPÄISCHE PATENTANMELDUNG

(11) **EP 4 378 447 A1**
(43) Veröffentlichungstag der Anmeldung: **05.06.2024**
(21) Anmeldenummer: 22210393.9
(22) Anmeldetag: 29.11.2022
(51) Int. Cl.: A61K 8/34, A61K 8/37, A61K 8/92, A61K 8/97, A61Q 19/00

(54) **KOSMETISCHE ZUSAMMENSETZUNG**

(71) Anmelder: Weleda AG, 4144 Arlesheim (CH)
(72) Erfinder: POST, Katharina, 4055 Basel (CH); MATZKE, Benjamin, 68220 Leymen (FR)
(74) Vertreter: Simandi, Claus

(57) **Zusammenfassung**

Die Erfindung betrifft eine kosmetische Zusammensetzung im Wesentlichen auf pflanzlicher Basis, welche nahezu wasserfrei ist, und bei der topischen Verwendung auf der Haut, vorzugsweise trockene Haut, in Kontakt mit Wasser eine angenehme Wärme erzeugt, sodass die Hautporen erweitert werden und Pflanzeninhaltsstoffe vorteilhaft in die Haut penetrieren können. Weiterhin betrifft die Erfindung die Herstellung einer solchen kosmetischen Zusammensetzung und deren Verwendung, insbesondere als Reinigungsprodukt oder Rinse-off Produkt.

## Beschreibung

Die Erfindung betrifft eine kosmetische Zusammensetzung im Wesentlichen auf pflanzlicher Basis, welche nahezu wasserfrei ist, und bei der topischen Verwendung auf der Haut, vorzugsweise trockene Haut, in Kontakt mit Wasser eine angenehme Wärme erzeugt, sodass die Hautporen erweitert werden und Pflanzeninhaltsstoffe vorteilhaft in die Haut penetrieren können. Weiterhin betrifft die Erfindung die Herstellung einer solchen kosmetischen Zusammensetzung und deren Verwendung, insbesondere als Reinigungsprodukt oder Rinse-off Produkt.

Weleda AG forscht mit modernen und in Verbindung mit anthroposophischen Methoden an vorteilhaften natürlichen Pflanzenstoffen für die Kosmetik.

Die Haut, insbesondere die Epidermis, ist als Barriereorgan des menschlichen Organismus in besonderem Maße äußeren Einwirkungen unterworfen (Kälte, Chemikalien, Stress, Umwelteinflüsse, etc.). Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Pflanzenwirkstoffe, insbesondere Sekundärmetabolite, können einen positiven Einfluss auf die Haut nehmen. Es bedarf jedoch schonender Mittel zur Pflege und Reinigung der Haut.

Der vorliegenden Erfindung liegt daher die Aufgabe zugrunde, eine kosmetische Zusammensetzung zur topischen Verwendung bereitzustellen, sodass wertvolle Pflanzeninhaltsstoffe vorteilhaft in die Haut penetrieren können und ein zusätzlicher Pflegeeffekt erreicht wird.

Überraschender Weise konnten die Erfinder feststellen, dass der Einsatz einer kosmetischen Zusammensetzung auf einer im Wesentlichen pflanzlichen Basis eines Ölgels bereitgestellt werden kann, welches bei Kontakt mit Wasser eine angenehme Wärme erzeugt, sodass die Hautporen erweitert werden können, und auf diese Weise vorteilhaft Pflanzeninhaltsstoffe in die Haut penetrieren können.

Die Aufgabe wird durch die technische Lehre von mindestens einem Patentanspruch gelöst. Insbesondere betrifft die Erfindung daher ein Ölgel, und zwar eine kosmetische Zusammensetzung enthaltend
a.) mindestes ein zwei-, drei oder vierwertiger Alkohol,
b.) mindestens ein Pflanzenöl,
c.) mindestens ein Fettsäuremonoester,
   wobei das Verhältnis von a.) : b.) : c.) in Gew. % 2-4 / 4-6 / 0,5 -1,5 beträgt,
d.) mindestes einen Emulgator,
e.) mindestens ein oder mehrere Pflanzeninhaltsstoffe, wobei mindestens ein sekundärer Pflanzenstoff vorliegt,
f.) maximal 3 Gew. % Wasser.

Gewichtsangaben beziehen sich auf 100 % einer erfindungsgemäßen kosmetischen Zusammensetzung. Eine solche 100 %-ige Ausführungsform kann dem Beispiel 1 entnommen werden.

In einer weiteren Ausführungsform der Erfindung ist bevorzugt, dass das Verhältnis von a.) : b.) : c.) in Gew. % 3 / 5 / 1 beträgt. Das erfindungsgemäße vorgenannte Verhältnis ist wesentlich für die geeignete Viskosität zum Erhalten eines homogenen Ölgels. Weiterhin ist die Viskosität durch die Verwendung eines Emulgators d.) zu optimieren. Werden jedoch die Ölgehalte zu sehr erhöht, wird eine zu hohe Festigkeit erreicht, welche sich nachteilig auf ein homogenes Fließverhalten auswirkt.

In einer weiteren Ausführungsform der Erfindung ist bevorzugt, dass das Pflanzenöl gemäß b.) mindestens zu 40 Gew. %, insbesondere zu 50 Gew. % in der kosmetischen Zusammensetzung enthalten ist.

Weiterhin soll der Wasseranteil, nicht mehr als 3 Gew. %, vorzugsweise 2 Gew. % betragen. Andernfalls liegt kein klares fließbares Ölgel vor, sondern Trübungen treten auf, sodass keine vorteilhafte Homogenität erreicht wird. Es soll jedoch durch den geringen Wasseranteil eine besondere Ölgel-Textur erreicht werden, welche fein verteilbar auf die Haut aufgetragen werden kann, so dass bei Kontakt mit Wasser ein maximales Wärmegefühl bzw. maximale Wärme entsteht.

Der erfindungsgemäße Emulgator wirkt sich einerseits auf die Viskosität des Ölgels aus, andererseits wird bei Kontakt mit Wasser eine vorteilhafte homogene Emulsion ausgebildet. Solche Emulgatoren können vorteilhaft aus der Gruppe der nichtionischen oder amphoteren Emulgatoren ausgewählt werden, wie Zuckerester, insbesondere vorzugsweise Sucroseester und Betaine. In einer weiteren Ausführungsform der Erfindung ist bevorzugt, dass der Emulgator gemäß d.)zu 1 - 6 Gew. %, insbesondere zu 2 - 4 Gew. % in der kosmetischen Zusammensetzung enthalten ist.

Nach der topischen Anwendung bzw. Auftragung auf der Haut wird bei Kontakt mit Wasser (z.B. Waschen) oder Befeuchten eine Emulsion unter angenehmer Wärmeentwicklung (negative Enthalpie) aus dem Ölgel ausgebildet, sodass die Hautporen erweitert werden können, und auf diese Weise vorteilhaft Pflanzeninhaltsstoffe in die Haut penetrieren können. Dies ist besonders vorteilhaft für die Anwendung als Reinigungsprodukt oder Rinse off Produkt, wobei das Abwaschen bzw. Entfernen unter Wärmeentwicklung erfolgt.

Das erfindungsgemäße Ölgel umfasst:
Zwei-, drei oder vierwertige Alkohole sind solche ausgewählt aus der Gruppe Ethandiol, Glycerin, Propandiol, Erythrit, Butandiol, C1-C4 Alkohole mit 2, 3 oder 4 OH-Gruppen. Glycerin ist erfindungsgemäß bevorzugt.

Pflanzenöl im Sinne dieser Erfindung bedeutet, dass ein Trioder Diglycerid vorliegt, wobei mindestens eine Fettsäure einoder mehrfach ungesättigt ist, insbesondere solche Fettsäuren mit 14 bis 26 C-Atomen, insbesondere Ölsäure, Linolsäure oder Linolensäure. Das Pflanzenöl ist bei Raumtemperatur flüssig. Solche nativen Pflanzenöle können weitere Substituenten enthalten, wie OH, NH₂ u.a. Solche Pflanzenöle werden durch verschiedene Verfahren gewonnen, wie Pressen (Kaltpressen, Heißpressen), Extraktion mit Lösungsmitteln (Heptan, Petrolether; u. a.), Superfluid-Chromatographie, o.a.

Ein oder mehrere Pflanzenöle im Sinne dieser Erfindung können aus der Gruppe ausgewählt sein, wie nicht abschließend Açaíöl, Algenöl, Arganöl Avocadoöl, Babaçuöl, Borretschöl, Cupuaçu-Butter, Distelöl, Erdnussöl, Haselnussöl, Hanföl, Jatrophaöl, Jojobaöl, Kakaobutter, Kokosöl, Kürbiskernöl, Leinöl, Macadamiaöl, Maiskeimöl, Mandelöl, Mangobutter, Marillenkernöl, Nachtkerzenöl, Olivenöl, Palmöl, Palmkernöl, Papayaöl, Pistazienöl, Pekannussöl, Perillaöl, Rapsöl, Reisöl, Rizinusöl, Senföl, Sesamöl, Sheabutter, Sojaöl, Sonnenblumenöl, Tungöl, Walnussöl, Wassermelonensamenöl, Traubenkernöl, Zedernöl.

Ein Fettsäuremonoester im Sinne dieser Erfindung bedeutet, dass eine Fettsäure mit einem Alkohol verestert ist, sodass der Fettsäuremonoester vorzugsweise 16-26 C-Atome aufweist, auch Esteröl genannt, insbesondere Isoamyllaurat ist bevorzugt. Weiterhin kann die Fettsäure verzweigt oder unverzweigt, ungesättigt oder gesättigt sein, jedoch ist gesättigt bevorzugt. Weiterhin ist bevorzugt, dass der Alkohol mehr als 1C-Atom umfasst, verzweigt oder unverzweigt ist, wie z.B. iso-Propanol, iso-Butanol, iso-Pentanol und ggfs. mehr als eine OH-Gruppe aufweist, wie z.B. 1,2-Pentandiol. Solche Fettsäuremonoester können aus Pflanzenölen hergestellt werden. Pflanzeninhaltsstoffe sind erfindungsgemäße solche die sekundäre Pflanzenstoffe enthalten oder aus diesen bestehen. Sekundäre Pflanzenstoffe, bzw. Sekundärmetaboliten leiten sich von Produkten des anabolen und katabolen Stoffwechsels ab, insbesondere von Kohlenhydraten und Aminosäuren. Erfindungsgemäße sekundäre Pflanzenstoffe umfassen insbesondere phenolische, isoprenoide oder alkaloide Verbindungen, wie Phenole, Polyphenole, Flavonoide, Kaffeesäurederivate, Xanthone, Terpene, Steroide u.a. Naturstoffe. Solche Pflanzeninhaltsstoffe können wässrige und / oder ethanolische, oder ölige Auszüge / Extrakte aus einem Pflanzenmaterial sein, insbesondere aus Blättern, Wurzeln, Samen, Blüten. Beispielsweise können solche sekundäre Pflanzenstoffe in ätherischen Pflanzenölen vorliegen (z.B. Terpene, etc.). Solche Pflanzenmaterialien können ausgewählt sein aus der Gruppe der Pflanzengattungen Aloe, Althaea, Angelica, Arnika, Artemisia, Calendula, Cannabis, Capsicum, Carum, Citrus, Centella, Echinacea, Hedeara, Humulus, Iberis, Iris, Juglans, Lepidium, Matricaria, Melissa, Mentha, Basilicum (Ocimum), Passiflora, Pelargonium, Primula, Punica, Quercus, Rosmarinus, Rumex, Salix, Salvia, Sambucus, Strychnos, Thymus, Vaccinium, Valeriana, Vebena, Viola, Vitex, Vitis.

In einer weiteren Ausführungsform der Erfindung ist bevorzugt, dass ein oder mehrere Pflanzeninhaltsstoff(e), wobei mindestens ein sekundärer Pflanzenstoff vorliegt gemäß e.)zu 0,5 - 3 Gew. %, insbesondere zu 1 - 2 Gew. % in der kosmetischen Zusammensetzung enthalten ist.

Die erfindungsgemäßen kosmetischen Zusammensetzungen können weiterhin kosmetische Hilfsstoffe enthalten, wie sie üblicherweise in solchen Zubereitungen verwendet werden, z. B. Antioxidantien, Radikalfänger, Konservierungsmittel, Bakterizide, Parfüme, Farbstoffe, Pigmente, die eine färbende Wirkung haben, Verdickungsmittel, oberflächenaktive Substanzen, weichmachende, oder andere übliche Bestandteile einer kosmetischen Formulierung wie Schaumstabilisatoren, Elektrolyte, u.a.

Nachfolgende Ausführungsbeispiele und Figuren dienen zur Erläuterung der Erfindung, ohne die Erfindung auf diese Beispiele zu beschränken.

### Beispiel 1:

Beispielhafte Zusammensetzung einer erfindungsgemäßen Zusammensetzung:

| **Inhaltsstoff** | **Funktion** | **Gewicht (Gew. %)** |
|---|---|---|
| Glycerin | 3-wertiger Alkohol | **30 Gew. %** |
| Aqua | | **2 Gew %** |
| Sucrosestearat | Emulgator | **2 Gew %** |
| Betain | Emulgator | **3 Gew %** |
| Sonnenblumenöl | Pflanzenöl | **50 Gew %** |
| Isoamyllaurat | Fettsäureester (Esteröl) | **10 Gew %** |
| Rosmarin, Kamille, Calendula, Viola | Pflanzenmaterial | **1 Gew %** |
| Parfum | Duftstoff | **1 Gew %** |
| Zusatzstoff(e) | | **1 Gew %** |

### Beispiel 2:

Herstellung einer erfindungsgemäßen Zusammensetzung:
Phase 1: Glycerin, wasserlöslicher oder
   glycerinlöslicher Extrakt (Pflanzeninhaltsstoffe) und Wasser gut vermischen, dann Feststoffe, wie den Emulgator und etwaige Zusatzstoffe vormischen und in die Phase 1 eingeben, homogenisieren und rühren bis eine homogene Mischung erhalten wird.
Phase 2: Pflanzenöle, Esteröle und öllösliche Extrakte (Pflanzeninhaltsstoffe) homogen vermischen, und auf 70-75°C erwärmen.

Abschließend Phase 1 langsam zu der Phase 2 dosieren, homogenisieren und auf 35°C abkühlen, dann ggf. Parfüm und etwaige Zusatzstoffe hinzufügen, erneut homogenisieren und auf < 30°C abkühlen.

Je nach Löslichkeit werden die Pflanzeninhaltsstoffe in Phase 1 oder Phase 2 hinzugegeben.

## Patentansprüche

1. Kosmetische Zusammensetzung enthaltend
a.) mindestes ein zwei-, drei oder vierwertiger Alkohol,
b.) mindestens ein Pflanzenöl,
c.) mindestens ein Fettsäuremonoester,
wobei das Verhältnis von a.) : b.) : c.) in Gew. % 2-4 / 4-6 / 0,5 -1,5 beträgt,
d.) mindestes einen Emulgator,
e.) mindestens ein oder mehrere Pflanzeninhaltsstoffe, wobei mindestens ein sekundärer Pflanzenstoff vorliegt,
f.) maximal 3 Gew. % Wasser.

2. Kosmetische Zusammensetzung nach Anspruch 1, wobei der Alkohol gemäß a.) ausgewählt ist aus der Gruppe Ethandiol, Glycerin, Propandiol, Erythrit, Butandiol, C1-C4 Alkohole mit 2, 3 oder 4 OH-Gruppen.

3. Kosmetische Zusammensetzung nach einem der vorstehenden Patentansprüche, wobei das Pflanzenöl gemäß b.) ausgewählt ist aus der Gruppe Açaíöl, Algenöl, Arganöl Avocadoöl, Babaçuöl, Borretschöl, Cupuaçu-Butter, Distelöl, Erdnussöl, Haselnussöl, Hanföl, Jatrophaöl, Jojobaöl, Kakaobutter, Kokosöl, Kürbiskernöl, Leinöl, Macadamiaöl, Maiskeimöl, Mandelöl, Mangobutter, Marillenkernöl, Nachtkerzenöl, Olivenöl, Palmöl, Palmkernöl, Papayaöl, Pistazienöl, Pekannussöl, Perillaöl, Rapsöl, Reisöl, Rizinusöl, Senföl, Sesamöl, Sheabutter, Sojaöl, Sonnenblumenöl, Tungöl, Walnussöl, Wassermelonensamenöl, Traubenkernöl, Zedernöl.

4. Kosmetische Zusammensetzung nach einem der vorstehenden Patentansprüche, wobei das Fettsäuremonoester gemäß c.) 16-26 C-Atome aufweist, verzweigt oder unverzweigt ist, ungesättigt oder gesättigt ist, insbesondere Isoamyllaurat ist.

5. Kosmetische Zusammensetzung nach einem der vorstehenden Patentansprüche, wobei das Verhältnis von a.) : b.) : c.) in Gew. % 3 / 5 / 1 beträgt.

6. Kosmetische Zusammensetzung nach einem der vorstehenden Patentansprüche, wobei das Pflanzenöl gemäß b.) mindestens zu 40 Gew. %, insbesondere zu 50 Gew. % enthalten ist.

7. Kosmetische Zusammensetzung nach einem der vorstehenden Patentansprüche enthaltend mindestens ein oder mehrere Pflanzeninhaltsstoffe, wobei mindestens ein sekundärer Pflanzenstoff vorliegt, ausgewählt aus der Gruppe phenolische, isoprenoide oder alkaloide Verbindungen, Phenole, Polyphenole, Flavonoide, Kaffeesäurederivate, Xanthone, Terpene, Steroide oder aus wässrigen und / oder ethanolischen, oder öligen Auszügen / Extrakte aus einem Pflanzenmaterial.

8. Kosmetische Zusammensetzung nach Anspruch 7, wobei das Pflanzenmaterial ausgewählt ist aus der Gruppe Aloe, Althaea, Angelica, Arnika, Artemisia, Calendula, Cannabis, Capsicum, Carum, Citrus, Centella, Echinacea, Hedeara, Humulus, Iberis, Iris, Juglans, Lepidium, Matricaria, Melissa, Mentha, Basilicum (Ocimum), Passiflora, Pelargonium, Primula, Punica, Quercus, Rosmarinus, Rumex, Salix, Salvia, Sambucus, Strychnos, Thymus, Vaccinium, Valeriana, Vebena, Viola, Vitex, Vitis.

9. Kosmetische Zusammensetzung nach einem der vorstehenden Patentansprüche, wobei der mindestens eine Emulgator gemäß d.) ausgewählt ist aus nichtionischen oder amphoteren Emulgatoren, insbesondere Zuckerester, insbesondere Sucroseester und Betaine und der Emulgator gemäß d.)zu 1 - 6 Gew. %, insbesondere zu 2 - 4 Gew. % enthalten ist.

10. Kosmetische Zusammensetzung nach einem der vorstehenden Patentansprüche aufweisend:
| | **Inhaltsstoff** | **Gewicht (Gew. %)** |
|---|---|---|
| 1.) | Glycerin | **30 Gew. %** |
| 2.) | Aqua | **2 Gew %** |
| 3.) | Sucrosestearat | **2 Gew %** |
| 4.) | Betain | **3 Gew %** |
| 5.) | Sonnenblumenöl | **50 Gew %** |
| 6.) | Isoamyllaurat | **10 Gew %** |
| 7.) | Extrakte Rosmarin und / oder Kamille und / oder Calendula und / oder Viola | **1 Gew %** |
| 8.) | Parfum | **1 Gew %** |
| 9.) | Andere kosmetische Zusatzstoffe | **1 Gew. %** |

11. Oelgel enthaltend eine kosmetische Zusammensetzung nach einem der Ansprüche 1 bis 9, welches nach Kontakt mit Wasser eine Emulsion unter Wärmeentwicklung ausbildet.

12. Rinse-Off Produkt enthaltend ein Ölgel nach Anspruch 11.
